(19) Europäisches Patentamt
European Patent Office
Office européen des brevets

(11) Veröffentlichungsnummer : **0 257 399 B1**

# (12) EUROPÄISCHE PATENTSCHRIFT

(45) Veröffentlichungstag der Patentschrift :
**02.01.91 Patentblatt 91/01**

(51) Int. Cl.[5] : **A61K 7/13**

(21) Anmeldenummer : **87111380.9**

(22) Anmeldetag : **06.08.87**

## (54) Haarfärbemittel.

(30) Priorität : **13.08.86 DE 3627434**
**03.12.86 DE 3641282**

(43) Veröffentlichungstag der Anmeldung :
**02.03.88 Patentblatt 88/09**

(45) Bekanntmachung des Hinweises auf die Patenterteilung :
**02.01.91 Patentblatt 91/01**

(84) Benannte Vertragsstaaten :
**AT BE CH DE ES FR GB GR IT LI LU NL SE**

(56) Entgegenhaltungen :
**DE-A- 3 434 468**
**FR-A- 1 274 158**
**US-A- 3 041 244**
**US-A- 4 021 538**

(56) Entgegenhaltungen :
**CHEMICAL ABSTRACTS, Band 80, 1974, Seite 237, Zusammenfassung Nr. 74265p, Columbus, Ohio, US; & JP-A-73 24 259**
**PATENT ABSTRACTS OF JAPAN, Band 1, Nr. 29 (C-76)[1549], 28. März 1977; & JP-A-51 148 043**

(73) Patentinhaber : **Beratungslabor für die kosmetische und pharmazeutische Industrie**
**Dipl.-Ing. Karlheinz Schrader**
**Max-Planck-Strasse 6**
**D-3450 Holzminden (DE)**

(72) Erfinder : **Schrader, Karlheinz**
**Im Erlengrund 16**
**D-3454 Bevern (DE)**

(74) Vertreter : **Patentanwälte Zellentin & Partner**
**Zweibrückenstrasse 15**
**D-8000 München 2 (DE)**

EP 0 257 399 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Haarfärbemittel mit verbesserten Gebrauchseigenschaften.

Die im Handel befindlichen Haarfärbemittel gliedern sich in zwei unterschiedliche Kategorien :

Einerseits die sogenannten permanenten Haarfärbemittel auf Basis von Oxidations-Farbstoffen bzw. Farbstoff-Vorprodukten, die nach Mischen mit Oxidationsmitteln, insbesondere Wasserstoffperoxid, zumeist in Gegenwart von Nuanceuren und Entwicklersubstanzen, zur Einwirkung gebracht werden.
Mit Hilfe dieser Oxidationsfärbemittel kann eine längere Zeit anhaltende Färbung des Haares erzielt werden.
Die Anwendung dieser Farbstoffprodukte durch den Verbraucher ist insofern umständlich, als Färbemittel bzw. Farbstoffvorprodukte einerseits und Peroxid andererseits bis zur Anwendung getrennt gehalten werden müssen, um eine vorzeitige Reaktion zu vermeiden.
Eine zweite Gruppe von im Einsatz befindlichen Haarfärbemitteln weist diesen Nachteil nicht auf, da sie zur Farbentwicklung nicht der Einwirkung von Oxidationsmitteln bedürfen ; dies sind die sogenannten semipermanenten Haarfärbemittel auf Basis direktziehender Farbstoffe.
Diese Zusammensetzungen können in einer Verpackung untergebracht werden und sind daher für den Verbraucher leicht zu handhaben. Sie weisen jedoch den Nachteil auf, daß lediglich eine oberflächliche Färbung des Haares erzielt wird, die nur kurze Zeit anhält und in ihrer Intensität mit derjenigen durch Oxidationsfarbstoffe erzielten nicht zu vergleichen ist.

Es bestand daher die Aufgabe, ein Haarfärbemittel zu entwickeln, das die Vorteile einer intensiven Haarfärbung durch Oxidationsfarbstoffe aufweist, jedoch die Nachteile der umständlichen Handhabung nicht mehr besitzt.

Diese Aufgabe wird erfindungsgemäß dadurch gelöst, daß ein Haarfärbemittel, das mindestens einen Oxidationsfarbstoff, mindestens ein Tensid und ggf. weitere Kuppler- und Entwicklersubstanzen enthält, als Suspension oder Paste lediglich in einer Verpackungseinheit vorliegt, im wesentlichen wasserfrei ist und mindestens ein Peroxid enthält, das vom Oxidationsfarbstoff vor der Anwendung nicht getrennt gehalten wird.

Durch dieses Mittel ist es möglich, ohne vorherigen separaten Mischvorgang von Peroxid und Oxidationsfarbstoff eine direkte Applikation auf das nasse Haar vorzunehmen.

Aus der DE-OS 2 005 074 ist bereits ein Haarfärbemittel bekannt, das als Aerosol appliziert werden soll und dadurch gekennzeichnet ist, daß ein Teil der Farbstoffe, u. a. Oxidationsfarbstoffe, in mikroverkapselter Form in Suspension in einem flüssigen, insbesondere wäßrigen Medium vorliegen.
Da in diesem Mittel auch die Peroxide in mikroverkapselter Form vorliegen sollen, kann das Produkt in einer Verpackungseinheit konfektioniert werden und bedarf bei der Anwendung ebenfalls keiner Vormischung.

Diese bekannte Lösung ist jedoch aus mehreren Gründen unzweckmäßig :

Zum einen ist die Mikroverkapselung ein sehr kostenintensives Verfahren, so daß die solchermaßen hergestellten Haarfärbemittel für den Verbraucher nahezu unerschwinglich wären. Dies dürfte auch der Grund dafür sein, daß derartige Produkte nicht auf den Markt gelangt sind, da sie offenbar als nicht wettbewerbsfähig angesehen werden.

Darüberhinaus ist der Einsatz mikroverkapselter Wirkstoffe deshalb problematisch, weil die Kapseln einerseits so stabil sein müssen, daß sie während der Herstellung und Abfüllung der jeweiligen Mittel nicht aufbrechen und den Inhalt vorzeitig freigeben, was insbesondere bei Haarfärbemitteln schon beim vorzeitigen Zerstören eines kleinen Teils von Peroxid bzw. Oxidationsfarbstoff äußerst nachteilige Folgen im Sinne einer Farbveränderung hervorrufen würde.

Andererseits müssen die Kapseln bei der Anwendung auf dem Haar sofort und vollständig aufbrechen, da sonst eine befriedigende Haarfärbung nicht möglich ist.

US-A-A-3041244 beschreibt pulverförmige wasserfreue Mischungen zu Haarfärbung, enthaltend Oxydationsfarbstoff und Peroxid. Diese müssen jedoch vor der eigentlichen Anwendung auf dem Haar mit Wasser eigens angeknetet werden.Dasselbe gilt für die in Chem.Abstracts, Band 80, 1974, S.237, Nr.74265 p, Patent Abstracts of Japan, 1549, C 76, NR. 51-148043 und DE-A-3434468 beschriebenen Produkte.

Die erfindungsgemäßen Zusammensetzungen können sowohl als Suspension bzw. Paste in üblichen Verpackungsmaterialien wie Tuben als auch als Aerosol-Zusammensetzungen konditioniert sein.

Sie enthalten vorzugsweise ein oder mehrere Verdickungsmittel, insbesondere Salze der Polyacrylsäure, Cellulosederivate, Alkali- und Erdalkalialuminiumsilikate, Bentonite wie Montmorillonit, kolloidales Siliciumdioxid, etc..
Die geeignete Menge dieser Stoffe liegt bei etwa 0,2 bis etwa 20, insbesondere etwa 1 bis 10, vorzugsweise 1,5 bis 5 Gew.-% der Gesamtzusammensetzung.
Die erfindungsgemäßen Haarfärbemittel können auch ein Lösungs- bzw. Verdünnungsmittel enthalten. Als solches ist jede physiologisch verträgliche, nichtwässrige Flüssigkeit geeignet ; bevorzugt sind kosmetische Fette und Öle sowie insbesondere Diole und deren Derivate wie 1,2-Propandiol, 1,3-Butandiol und 1-Methoxypropanol(-2) und deren Gemische.

Diese Stoffe werden in einer Menge von etwa 10 bis etwa 80, vorzugsweise 20 bis 50, insbesondere 25 bis 40 Gew.-% der Gesamtzusammensetzung eingesetzt.

Den erfindungsgemäßen Haarfärbemitteln können alle in solchen Mitteln üblichen Stoffe zugesetzt werden.

Neben den obligatorischen Tensiden sind dies insbesondere Reduktionsmittel wie Natriumsulfit oder Ascorbinsäure und Stoffe mit konditionierender Wirkung.

Als solche sind vor allem die verschiedenen Polymeren, vorzugsweise solche mit quaternären Ammoniumgruppen, zu nennen wie quaternäre Celluloseether vom Typ "Polymer JR"® oder quaternierte synthetische Polymere vom Typ "Merquat"®.

Geeignete Tenside zum Einsatz in den erfindungsgemäßen Haarfärbeund -tönungsmitteln sind Alkylsulfate, Alkansulfonate, Fettsäureamidsulfate, Alkylethercarbonsäuren, Fettsäuresarkosinate, Isethionate und Gemische aus den genannten Substanzen, insbesondere in Form ihrer Alkali- oder Aminsalze.

Verwendet werden können auch nichtionische Tenside wie Fettsäuremono-und -dialkylolamide, Fettalkoholpolyglykolether, Aminoxide, ampholytische Tenside wie Betaine, Sulfobetaine und N - Alkylaminocarbonsäuren.

Tenside, die im Rahmen der Erfindung bevorzugt eingesetzt werden können, sind Ethylenoxid/Propylenoxid-Mischkondensate vom Typ Pluronic®, die zusätzlich konsistenzregelnde Eigenschaften aufweisen.

Die Tenside sind in den erfindungsgemäßen Mitteln in einer Menge von etwa 0,5 bis etwa 80 Gew.-%, vorzugsweise 5 bis 50, insbesondere 10 bis 30 Gew.-%, enthalten. Die zweckmäßige Konzentration an Tensiden ist auch von deren Aggregatzustand (fest oder flüssig) abhängig.

Als Oxidationsfarbstoff-Vorprodukte sind insbesondere Phenylendiamine und deren Derivate geeignet, beispielsweise p-Phenylendiamin, m-Phenylendiamin und ihre N-substituierten Derivate und Salze, N-substituierte Derivate des o-Phenylendiamins, o-und p-Toluylendiamin und ihre N-substituierten Derivate bzw. Salze sowie N-substituierte Derivate des m-Toluylendiamins, Aminokresole und Diaminokresole und deren Methyl-Derivate.

Eine als Oxidationsfarbstoff-Vorprodukte geeignete Substanzklasse sind auch die Aminopyridine, beispielsweise 2,5-Diaminopyridin, 2,5-Diamino-4-methylpyridin, 2-(2-Hydroxyethylamino)-5-aminopyridin, 2-Phenylamino-5-aminopyridin, 2,6-Diaminopyridin, 2,6-Dihydroxy-3-aminopyridin, 2-Bis(2-hydroxyethylamino)-5-aminopyridin, 3-Amino-2-methylamino-6-methoxypyridin, 3-Amino-2-(2-hydroxyethylamino)-6-methoxypyridin, 2,6-Dihydroxypyridin, 2,6-Dimethoxy-3,5-diaminopyridin, 2-Diethylamino-3,5-diaminopyridin und 2-(2-Hydroxyethylamino)-3-amino-6-methoxypyridin, vorzugsweise in Form ihrer Salze wie der Hydrochloride.

Weitere geeignete Pyridin-Derivate sind beispielsweise 3-Amino-6-(dimethylamino)pyridin, 3-Amino-6-methoxy-2-phenylaminopyridin, 3,5-Diamino-2-(N,N-diethylamino)pyridin und 2-(-Propylamino)-5-aminopyridin.

Besonders geeignet sind auch Nitropyridine wie N-(2-Hydroxyphenyl)-6-methoxy-3-nitropyridin, 2-(N,N-Dimethylamino)-6-ethoxy-3-nitropyridin, 2-(Methylamino)-6-ethoxy-3-nitropyridin, 2-(3-Dimethylaminopropylamino)-5-nitropyridin, 2-(4-Methoxyphenylamino)-6-methoxy-3-nitropyridin, 2-(2-Aminoethylamino)-6-methoxy-3-nitropyridin, 2-(2-Hydroxyethylamino)-6-methoxy-3-nitropyridin, 2,6-Diamino-3-nitropyridin, 2,6-Bis-(2-hydroxyethylamino)-3,5-dinitropyridin, 2-(2-Aminomethylamino)-5-nitropyridin, 2-((2-Hydroxyethyl)-methylamino)-5-nitropyridin, 2-(2-Aminoethylamino)-3-nitropyridin und 3,5-Dinitro-6-methoxy-2-aminopyridin, insbesondere in Form ihrer Hydrochloride.

Die erfindungsgemäßen Haarfärbemittel können, neben den Oxidationsfarbstoff-Vorprodukten, weitere bekannte Entwickler-und Kupplersubstanzen sowie Nuanceure enthalten.

Als solche sein insbesondere o-, m- und p-Aminophenol, 1-Methyl-2-hydroxy-4-aminobenzol, 1,2-Dihydroxybenzol, Resorcin und 2-bzw. 4-Chlorresorcin, 2,5-Diaminoanisol, a-Naphtol, Diphenylamin und dessen Derivate, Pyrogallol, 1-Hydroxy-2,4-diaminobenzol, Picraminsäure, 6-Amino-3-methylphenol und Hydrochinon genannt.

Außer den obengenannten ist im Prinzip jeder Oxidationshaarfarbstoff, ggf. in Kombination mit Entwicklern, Kupplern und Nuanceuren, zur Herstellung der gewünschten Farbtöne geeignet. Möglich und in manchen Fällen zweckmäßig ist auch die Mitverwendung direktziehender Farbstoffe.

Als Peroxide werden in den erfindungsgemäßen Zusammensetzungen vorzugsweise Benzoylperoxid, Harnstoffperoxid, Cumolhydroperoxid und insbesondere Melaminperoxid eingesetzt, jedoch kann im Prinzip jedes geeignete Peroxid oder auch ein Gemisch aus Peroxiden Verwendung finden.

Ein komplexes Addukt aus Wasserstoffperoxid, Natriumsulfat und Kaliumchlorid mit der empirischen Formel

$$2\ H_2O_2 \cdot 4\ Na_2SO_4 \cdot KCl$$

eignet sich gut als peroxidische Oxidationskomponente in den erfindungsgemäßen Haarfärbemitteln.

Durch die Verwendung dieses Peroxids wird eine schnelle, vollständige Oxidation des Oxidationsfarbstoff-Vorproduktes bei der Anwendung des erfindungsgemäßen Mittels erreicht ; andererseits bleibt diese Peroxidverbindung während der Lagerung des Mittels außergewöhnlich stabil.

Die Menge an eingesetztem Peroxid beträgt, ausgedrückt als Wasserstoffperoxid-Gehalt, etwa 2 bis etwa 10, vorzugsweise 3 bis 8, insbesondere 4 bis 6 % $H_2O_2$.

Als Alkalisierungsmittel können insbesondere Alkaliphosphate wie Trinatriumorthophosphat mitverwendet werden, insbesondere in Kombination mit Ammoniumsalzen wie Ammoniumsulfat oder Ammoniumchlorid.

Die Anwendung der erfindungsgemäßen Zusammensetzung zum Haarfärben erfolgt durch das Auftragen und gleichmäßige Verteilen der Mischung auf mit Wasser kräftig gewässertes Haar und Einwirkenlassen in an sich bekannter Weise.

Die folgenden Beispiele dienen der Illustration der Erfindung.

## Beispiel 1

| | | |
|---|---|---|
| p-Toluylendiaminsulfat | | 1,0 (Gew.-%) |
| Resorcin | | 0,5 |
| p-Aminophenolhydrochlorid | | 0,2 |
| 1,2-Propandiol | | 10,0 |
| Ammoniumsulfat | | 6,0 |
| Trinatriumorthophosphat | | 10,0 |
| Melaminperoxid | | 10,0 |
| Kolloidales Siliciumdioxid | | 1,0 |
| Natriumdithionit | | 0,1 |
| Fettalkoholpolyglykolether | ad. | 100,0 |

Diese Mischung kann in eine Tube abgefüllt oder im Innenbehälter einer Zweikammeraerosoldose, deren Inhalt separat vom Treibmittel gehalten wird, beispielsweise nach der DE-OS 2 705 549, untergebracht werden.

Beim Aufbringen auf nasses Haar wird nach 20-30 minütiger Einwirkung und anschließender Wäsche ein braunroter Farbton erhalten

## Beispiel 2

| | |
|---|---|
| p-Toluylendiaminsulfat | 3,0 (Gew.-%) |
| Resorcin | 0,1 |
| p-Aminophenolhydrochlorid | 1,0 |
| m-Aminophenol | 0,12 |
| Vaseline | 23,0 |
| Ammoniumsulfat | 6,0 |
| Trinatriumorthophosphat | 6,0 |
| Melaminperoxid | 10,0 |
| Natriumdithionit | 0,2 |
| Polyoxyethylenlaurylether | ad. 100,00 |

Nach der Haarbehandlung analog Beispiel 1 wird ein hellbrauner Farbton erhalten.

## Beispiel 3

| | |
|---|---|
| p-Toluylendiaminsulfat | 1,0 (Gew.-%) |
| m-Aminophenol | 0,5 |
| Kolloidales Siliciumdioxid | 2,5 |
| Trinatriumorthophosphat | 12,0 |
| Ammoniumsulfat | 10,0 |
| Natriumdithionit | 0,05 |
| EDTA, Tetranatriumsalz | 0,5 |
| Melaminperoxid | 16,0 |
| Ethylenoxid/Propylenoxid-Kondensat (Pluronic[R] L 64) | ad 100,0 |

Dieses Mittel ergibt bei der Haarfärbung einen satten Braunton.

Beispiel 4

| | |
|---|---|
| p-Toluylendiaminsulfat | 1,0 (Gew.-%) |
| m-Phenylendiamin | 0,5 |
| Kolloidales Siliciumdioxid | 2,0 |
| Trinatriumorthophosphat | 12,0 |
| Ammoniumsulfat | 10,0 |
| Melaminperoxid | 10,0 |
| EDTA, Tetranatriumsalz | 0,5 |
| Natriumdithionit | 0,2 |
| Ethylenoxid/Propylenoxid-Mischkondensat | ad 100,0 |

Mit dieser Färbemischung kann menschlichem Haar ein blauer Farbton verliehen werden.

Beispiel 5

| | |
|---|---|
| p-Toluylendiaminsulfat | 1,0 (Gew.-%) |
| m-Aminophenol | 0,5 |
| Kolloidales Siliciumdioxid | 2,06 |
| Trinatriumorthophosphat | 12,0 |
| Ammoniumsulfat | 10,0 |
| EDTA, Tetranatriumsalz | 0,5 |
| Melaminperoxid | 16,0 |
| Natriumdithionit | 0,1 |
| Antioxidans | 0,1 |
| Ethylenoxid/Propylenoxid-Mischkondensat | ad 100,0 |

Diese Farbmischung ergibt eine Braunfärbung.

Beispiel 6

| | |
|---|---|
| 2,5-Diaminopyridinhydrochlorid | 1,0 (Gew.-%) |
| 2,6-Bis(2-hydroxyethylamino)-2,5-dinitropyridinhydrochlorid | 0,5 |
| Decyloleat | 10,5 |
| Fettalkoholpolyglykolether | 21,0 |
| Trinatriumorthophosphat | 7,5 |
| Ammoniumsulfat | 6,5 |
| Kolloidales Siliciumdioxid | 2,0 |
| Harnstoffperoxid | 10,0 |
| Natriumdithionit | 0,2 |
| Ethylenoxid/Propylenoxid-Mischkondensat (Pluronic$^R$ L 64) | ad 100,0 |

Mit dieser Farbmischung läßt sich eine kupferrote Haarfarbe erzielen.

Beispiel 7

| | |
|---|---|
| p-Toluylendiaminsulfat | 2,5 (Gew.-%) |
| 3-Amino-2-(2-hydroxyethylamino)-6-methoxypyridin | 0,3 |
| Resorcin | 0,3 |
| m-Aminophenol | 0,2 |
| 1-Methoxypropanol(-2) | 10,5 |
| Melaminperoxid | 10,0 |
| Trinatriumorthophosphat | 12,0 |
| Ammoniumsulfat | 10,0 |
| Natriumdithionit | 0,2 |
| Organisch modifizierter Montmorillonit (Bentone$^R$) | 2,0 |
| Fettalkoholpolyglykolether | ad 100,0 |

Die Haare können mit dieser Zusammensetzung tiefschwarz gefärbt werden.

Beispiel 8

| | |
|---|---|
| Trinatriumorthophosphat | 18,5 (Gewichtsteile) |
| Ammoniumsulfat | 15,6 |
| EDTA, Trinatriumsalz | 0,9 |
| 4 $Na_2SO_4$.2 $H_2O_2$.KCl (Oxycal[R]) | 60,0 |
| 1,2-Propylenglycol | 68,2 |
| Methylencellulose | 0,7 |
| Thioglycolsäure | 0,5 |
| Ethylenoxid/Propylenoxid-Copolymer | 22,0 |
| p-Toluylendiaminsulfat | 1,5 |
| p-Aminophenolhydrochlorid | 0,5 |
| 2-Chlorresorcin | 0,2 |
| m-Aminophenol | 0,2 |
| α-Naphtol | 0,1 |

Diese Mischung wird in den flexiblen Innenbehälter einer Zweikammer-Aerosoldose eingebracht, und die Dose in üblicher Weise mit Treibgas aufgefüllt.
Beim Aufbringen des Mittels auf nasses Haar wird eine dunkelbraune Färbung erzielt.

**Ansprüche**

1. Haarfärbemittel, enthaltend mindestens einen Oxidationsfarbstoff, sowie mindestens ein Tensid und ggf. weitere Kuppler- und Entwicklersubstanzen , dadurch gekennzeichnet, daß es als Suspension oder Paste in einer einzigen Verpackungseinheit vorliegt, im wesentlichen wasserfrei ist und mindestens ein Peroxid enthält, das vom Oxidationsfarbstoff vor der Anwendung nicht getrennt gehalten wird.

2. Haarfärbemittel nach Anspruch 1, dadurch gekennzeichnet, daß es zusätzlich mindestens ein Verdickungsmittel enthält.

3. Haarfärbemittel nach Anspruch 1 und/oder 2, dadurch gekennzeichnet, daß es zusätzlich mindestens ein Lösungs-oder Verdünnungsmittel enthält.

4. Haarfärbemittel nach Anspruch 3, dadurch gekennzeichnet, daß es als Lösungs- oder Verdünnungsmittel 1,2-Propandiol und/oder 1-Methoxypropanol(-2) enthält.

5. Haarfärbemittel nach einem der vorhergehenden Ansprüche, dadurch gekennzeichnet, daß es als Peroxid Melaminperoxid enthält.

6. Haarfärbemittel nach einem der vorhergehenden Ansprüche dadurch gekennzeichnet, daß es als Peroxid einen Komplex aus Wasserstoffperoxid, Natriumsulfat und Kaliumchlorid der empirischen Formel

$$2\,H_2O_2\,.\,4\,Na_2SO_4\,.KCl$$

enthält.

7. Haarfärbemittel nach Anspruch 6, dadurch gekennzeichnet, daß es den Peroxid-Komplex in einer Menge von 2 bis 10 Gew.% der Gesamtzusammensetzung, berechnet als Wasserstoffperoxid, enthält.

8. Haarfärbemittel nach einem der vorhergehenden Ansprüche, dadurch gekennzeichnet, daß es ein Ethylenoxid/Propylenoxid-Mischkondensat enthält.

9. Verfahren zum Färben menschlicher Haare, dadurch gekennzeichnet, daß auf das nasse Haar eine Zusammensetzung nach einem der vorhergehenden Ansprüche aufgetragen, gleichmäßig verteilt und in an sich bekannter Weise zur Einwirkung gebracht wird.

## Claims

1. Hair colorant containing at least one oxidation dye as well as at least one surfactant and optionally further coupler and developer substances, characterised in that it exists as a suspension or paste in a single packaging unit, is substantially anhydrous and contains at least one peroxide which is not kept separately from the oxidation dye prior to use.

2. Hair colorant according to claim 1, characterised in that it additionally contains at least one thickener.

3. Hair colorant according to claim 1 and/or 2, characterised in that it additionally contains at least one solvent or diluent,

4. Hair colorant according to claim 3, characterised in that it contains 1,2-propane diol and/or 1-methoxypropanol (-2) as solvent or diluent.

5. Hair colorant according to one of the preceding claims, characterised in that it contains melamine peroxide as a peroxide.

6. Hair colorant according to one of the preceding claims, characterised in that it contains, as a peroxide, a complex of hydrogen peroxide, sodium sulphate and potassium chloride corresponding to the empirical formula

$$2\ H_2O_2\ .\ 4\ Na_2SO_4\ .\ KC1.$$

7. Hair colorant according to claim 6, characterised in that it contains the peroxide complex in a quantity of 2 to 10% by weight of the total composition, calculated as hydrogen peroxide.

8. Hair colorant according to one of the preceding claims, characterised in that it contains an ethylene oxide/propylene oxide mixed condensate.

9. Process for dyeing human hair, characterised in that a composition according to one of the preceding claims is applied to the wet hair, is distributed uniformly and is caused to act in a known manner.

## Revendications

1. Agent de teinture capillaire contenant au moins un colorant d'oxydation de même qu'au moins un agent tensio-actif, et, le cas échéant, d'autres substances de couplage et de développement, caractérisé par le fait qu'il se présente sous la forme d'une suspension ou d'une pâte dans une unité d'emballage unique, qu'il est sensiblement anhydre, et qu'il contient au moins un peroxyde qui n'est pas tenu séparé du colorant d'oxydation avant l'utilisation.

2. Agent de teinture capillaire selon la revendication 1, caractérisé par le fait qu'il contient en outre au moins un agent épaississant.

3. Agent de teinture capillaire selon la revendication 1 et/ou 2, caractérisé par le fait qu'il contient en outre au moins un solvant ou diluant.

4. Agent de teinture capillaire selon la revendication 3, caractérisé par le fait qu'il contient comme solvant ou diluant du propanediol-1,2 et/ou du méthoxy-1 propanol(-2).

5. Agent de teinture capillaire selon l'une des revendications précédentes, caractérisé par le fait qu'il contient du peroxyde de mélamine en tant que peroxyde.

6. Agent de teinture capillaire selon l'une des revendications précédentes, caractérisé par le fait qu'il contient,comme peroxyde, un complexe de peroxyde d'hydrogène, de sulfate de sodium et de chlorure de potassium de la formule empirique :

$$2\ H_2O_2\ .\ 4\ Na_2SO_4\ .\ KCl$$

7. Agent de teinture capillaire selon la revendication 6, caractérisé par le fait qu'il contient le complexe de peroxyde dans une quantité de 2 à 10% en poids de la composition totale, calculé en tant que peroxyde d'hydrogène.

8. Agent de teinture capillaire selon l'une des revendications précédentes, caractérisé par le fait qu'il contient un co-condensat oxyde d'éthylène-oxyde de propylène.

9. Procédé de teinture des cheveux humains, caractérisé par le fait qu'on répartit uniformément sur les cheveux mouillés une composition selon l'une des revendications précédentes et qu'on fait agir d'une manière connue en soi